# EUROPÄISCHE PATENTANMELDUNG

(11) **EP 1 564 556 A1**
(43) Veröffentlichungstag der Anmeldung: **17.08.2005**
(21) Anmeldenummer: 04003497.7
(22) Anmeldetag: 17.02.2004
(51) Int. Cl.: G01N 33/543

(54) **Verfahren und Vorrichtung zur Bestimmung mehrerer Analyten mit simultaner interner Kontrolle**

(71) Anmelder: DST Diagnostic Science & Technology GmbH, 19061 Schwerin (DE)
(72) Erfinder: Schwertner, Heiko, 19055 Schwerin (DE); Runge, Dorothee Monika, 19057 Schwerin (DE)
(74) Vertreter: Krauss, Jan

(57) **Zusammenfassung**

Die vorliegende Erfindung betrifft eine Vorrichtung, in der auf einer Oberfläche Moleküle immobilisiert werden, welche in der Lage sind, mit den nachzuweisenden Analyten zu reagieren, so daß diese gebunden werden und in einer nachfolgenden Reaktion oder mehreren Reaktionsschritten nachgewiesen werden können. Dabei werden mindestens zwei solcher Flächen grafisch zusammenhängend kombiniert und derart ausgestaltet, daß diese mit einer Probenmatrix gleichzeitig in Kontakt kommen, wobei eine der Flächen zum Nachweis eines Analyten und die andere zur Kontrolle oder zur Quantifizierung des Analyten dient. Die erfindungsgemäße Vorrichtung und ein entsprechendes Verfahren kann für alle diagnostischen Bereiche eingesetzt werden, insbesondere der medizinischen Diagnostik, wie Allergie-, Infektions-, Typisierungs, DNA/RNA- und pharmakologischer wie toxikologischer Diagnostik, aber auch der Lebensmittel-, der veterinärmedizinischen - oder der Umweltdiagnostik.

## Beschreibung

Die vorliegende Erfindung betrifft ein Vorrichtung zum Nachweis von Molekülen oder Molekülklassen oder Molekülgemischen, bei der auf einer Oberfläche der Vorrichtung mindestens zwei Flächen derart chemisch oder physikalisch modifiziert sind, daß diese mit Mitteln zur Immobilisierung von Molekülen oder Molekülklassen und/oder Gemischen versehen sind, wobei eine Fläche zur Kontrolle bzw. Standardisierung, und die andere zum Nachweis eines Analyten verwendet wird, wobei die beiden Flächen so aufgebaut sind, daß sie im wesentlichen zum gleichen Zeitpunkt mit einer gesamten Probenmatrix, aus der Molekülen oder Molekülklassen oder Molekülgemischen bestimmt werden sollen, in Kontakt kommen (Tangential-Touch-Test Prinzip), und wobei beide Flächen so aufgebaut und zueinander angeordnet sind, daß sie gemeinsam ausgewertet werden, wobei sie eine optisch auslesbare grafische Anordnung bilden. Erfindungsgemäß können als Symbolpaare - für negativ und + für positiv, oder Kreis für negativ und Kreis mit innen liegendem Punkt/Punkten für positiv sichtbar werden. Als Mittel zur Immobilisierung von Molekülen oder Molekülklassen und/oder Gemischen ausgewählt können Antikörper, Antigenen, DNA, RNA, Enzymen, Substraten, Rezeptoren, Liganden und Kombinationen davon vorgesehen sein.

### Hintergrund der Erfindung

In der Forschung, Medizin, Biologie, Chemie, Toxikologie oder einer Vielzahl weiterer Anwendungsfelder stellen analytische Labortests, die zur qualitativen oder/und quantitativen Bestimmung von Molekülen bzw. deren Aktivität oder Zusammensetzung dienen, die Basis für weitreichende Aussagen bis hin zur Entwicklung neuer Verfahren oder Vorrichtungen dar. Beispiel hierfür sind die analytischen Methoden der Molekularbiologie, die zum einen in der Forschung, in forensischen Verfahren zur Aufklärung von Verbrechen oder in der Medizin zur Entdeckung von Krebserkrankungen eingesetzt werden. Die Basis sind die allgemein bekannten Methoden der DNA/RNA Analytik bzw. der Proteinanalytik. Ein weiteres Beispiel sind die Vielzahl von analytischen Verfahren und Methoden, die eingesetzt werden, um die Antikörperreaktionen, sogenannte Immunreaktionen, welche für die Bestimmung von Keimen, Proteinen, Drogen und vielen weiteren Substanzen eingesetzt werden. Diese haben einen der am weitesten gefaßten Anwendungsbereiche. Sie werden sämtlichen Gebieten der Medizin, Forschung, Lebensmitteltechnologie bis zum Bereich der Medikamentation eingesetzt.

Durch diese vielfältigen analytischen Einsatzmöglichkeiten von DNA/RNA, Proteinen und anderen Molekülen sind der Vergangenheit immer weiter die Labortechniken verfeinert worden. Dies führt zu immer größeren Erfolgen, was die Einsatzbereiche wieder erweitert hat. Dies führte in der Konsequenz zu immer größeren Probezahlen, welche gezielt auf bestimmte Moleküle untersucht werden. Da nur mit erheblichem Aufwand eine Vielzahl von einzelnen Proben untersucht werden können werden heute sogenannte Laborroboter eingesetzt oder in neuester Zeit sogenannte Chip-Technologien verwendet, welche in der Lage sind, simultan bis zu mehreren hundert Proben zu untersuchen.

Im Gegensatz zu diesen aufwendigen Laboruntersuchungsmethoden (EP 1 338 895 Titel: High density allergen microarray oder EP 0 875 758 B1 Titel: Immunoassay) werden immer häufiger sogenannte Schnelltests angewandt, welche dem Arzt oder Therapeuten eine schnelle, einfache und auf die wesentlichen Punkte beschränkte analytische Aussage liefern. Der Bedarf ist sehr groß und wächst stetig. Häufig werden diese Tests im sogenannten "Point of Care" Bereich, also unmittelbar vor, während oder nach therapeutischen Maßnahmen, eingesetzt. Ziel ist es auch, eine kostengünstige Alternative zum klassischen Labortest zu bieten. Allerdings ist die Sicherheit der Anwendung oder/und die Zahl der simultan bestimmbaren Analyten sowie der analytischen Genauigkeit, enge Grenzen gesetzt. Hier besteht erheblicher Verbesserungsbedarf.

Es sind Schnelltestverfahren bekannt, wie der Lateral-Flow-Test (LFT), Flow-Through-Test (FTT), Agglutinations-Test (AT) oder Solid-Phase-Test (SPT). Alle diese Verfahren dienen zum schnellen Nachweis von Analyten ohne die Verwendung von Geräten und sind zur visuellen Auswertung geeignet. Alle diese Verfahren haben Nachteile und sind somit nur bedingt Ersatz für Labortests bzw. können nur eingeschränkt durch Laien eingesetzt werden. Deshalb werden diese Verfahren zum Teil durch technische Hilfsmittel ergänzt. Beispiel für diese Verfahren sind u.a. die Patentschriften DE 197 21 151, EP 98 928 264.5, WO 98/53321 Titel: Streifentest zur in vitro Allergiediagnostik; EP 1 369 391; WO96/10747 Titel: Device an method utilizing arrays of structures for analyte capture; WO 03/094716; US 2003-212316 Titel: Method an apparatus for determining blood paramters and vital signs of a patient; WO 02/084249 Titel: Therapeutic an diagnostic uses of antibody specificity profiles. WO 00/40967 Titel: Method and Device for diagnosing allergy Symptoms; WO 02/066602, EP 1350111; WO 93/10458 Binding of Milk allergens to a solid phase; US 6,528,325, WO02/056017 Titel: Method for the visual detection of specific antibodies in human serum by the use of lateral flow assays; EP 1327884 Titel: Reagent test strip comprising conttrol means and timer means; WO 97/31268 Chromatographic strip having detection andcontrol zones oriented paraell to the direction flow; US 6,040,195 Titel: Diagnostic sanitary test strip; US 6,509,196, WO 01/50129 Titel: Compensation for non-spcific signals in quantitative immunoassays.

Die LFT sind die am weitesten verbreiteten Schnelltests. Hier wird die Probe z.B. Serum, Urin, auf eine Fläche aufgetragen. Direkt vergleichbar mit einer Dünnschichtchromatographie wird die Flüssigkeit (Probe) durch eine Trennschicht mit Reagenzien z.B. markierten Antikörpern, weiter durch eine Nitrocelluloseschicht gesogen. In dieser befinden sich Fangschichten, (im Gegensatz zum SPT bei dem sich auf der Oberfläche des Trägers Fangschichten befinden) welche markierte Antikörper binden und eine visuell sichtbare Line bilden. Solche Verfahren sind in der wissenschaftlichen Literatur vor 1980 beschrieben, sowohl in der Anwendung als klassische Dünnschichtchromatographie, als auch als Schnelltest. Unterschiede finden sich nur in der Verwendung eines vereinheitlichten Reaktionsgefäßes oder einer universell und mehrfach verwendbaren Trennschicht.

Der FTT ist vergleichbar einer Säulenchromatographie. Hier fließt die Probe (Flüssigkeit) durch Schichten von Membranen und Saugschichten. Auf der Nachweismembran sind, wie beim LFT z.B.: Antikörper gebunden, welche in der Lage sind Analyten zu binden. Die gebundenen Analyten werden mittels verschiedener Nachweisreagenzien als Punkte sichtbar gemacht. Solche Verfahren sind in der wissenschaftlichen Literatur vor 1980 beschrieben, sowohl in der Anwendung als Säulenchromatographie als auch als Schnelltest. Unterschiede finden sich nur in der Verwendung eines Reaktionsgefäßes oder einer universell und mehrfach verwendbaren Säule.

Das AT-Verfahren basiert auf mit Antikörpern beschichteten Partikeln. Diese befinden sich in einer ebenmäßigen Schicht. Wird eine positive Proben hinzugegeben, so wird die ebenmäßige Schicht durch Quervernetzung zerstört, was bei starken Reaktionen mit bloßem Auge sichtbar ist.

Beim SPT-Verfahren, häufig auch als "dipstick"-Test bezeichnet, werden mittels Antikörpern oder Antigenen die Analyten auf einer Oberfläche gebunden (im Gegensatz zum LPT, bei denen in einer Oberfläche die Analyten gebunden werden) und anschließend durch nachfolgende Reaktionen nachgewiesen. Je nach verwendeter Nachweismethode, kann es zu einem mit bloßem Auge sichtbaren Punkt oder Fläche kommen. Ein solches Verfahren stellt das in der Patentschrift EP 98 928 264.5 (Titel: Streifentest zur in vitro Allergiediagnostik) beschriebene Verfahren dar. Allerdings sind solche Verfahren auch in der wissenschaftlichen Literatur vor 1980 beschrieben.

Alle Verfahren haben individuelle Vor- und Nachteile. Ein idealer Schnelltest sollte mit bloßem Auge auswertbar sein, eine Vielzahl von Analyten in einer Probe bestimmen können, über interne Standards zur Funktions-, Qualitätskontrolle und Mengenbestimmung verfügen und auch für einen Laien auswertbar sein. Ferner sollte der Test schnell, also ca. binnen 25 min oder weniger durchführbar und zugleich kostengünstig herzustellen sein.

LF-Tests können nur einzelne und nur in Ausnahmefällen mehrere Analyten in einer Probe bestimmen, da hier die Probenflüssigkeit durch die Membran fließen muß. Zwar ist hier eine positiv-negativ-Kontrolle möglich, nicht aber deren Quantifizierung. Gleiches gilt für die AT, FTT und SPT Tests, wobei meist auf einen externen Standard wie eine Farbkarte (siehe Patentschrift EP 98928264.5) oder ein Graumuster zurückgegriffen wird. Dies ist für eine visuelle Auswertung, also eine Auswertung mit bloßem Auge, negativ, da es hier leicht zu Fehlinterpretationen kommt. Ferner ist die Empfindlichkeit dadurch stark herabgesetzt. Die, seit den frühen 80'iger Jahren bekannten SPT, AT und FTT Verfahren bieten die Möglichkeit, eine Vielzahl von Analyten in einem Raster (Matrix) vergleichbar den späteren Chip-Technologien im Laborbereich, zu bestimmen. Keines der Verfahren ist jedoch in der Lage, in jedem der Rasterpunkte z.B. mittels eines internen Standards, eine Funktions-, Qualitätskontrolle und Mengenbestimmung zur gewährleisten. Dies ist aber notwendig, da bei einer ungleichmäßigen Verteilung der Probenflüssigkeit über die Testoberfläche, es zu Konzentrationsunterschieden der zu bestimmenden Analyten über eben dieser Fläche kommt. Dies führt zwangsläufig zu Fehlinterpretationen. Eine Standardreihe mit oder ohne positiv bzw. negativ Kontrolle führt am Rande der Rasterpunkte oder gar durch externe Farbkarte oder Grauabstufung verstärkt diesen Effekt der Fehlinterpretation noch.

Es ist somit Aufgabe der vorliegenden Erfindung, die bestehenden Nachteile der zur Zeit vorherrschenden Vorgehensweise und deren Verfahren aus dem Bereich der Schnelltestverfahren zu vermeiden, ohne die Vorteile der klassischen Labortestverfahren inkl. Laborroboter und/oder Chip-Systemen zu verlieren. Ferner ist es das Ziel, die Testverfahren so zu vereinfachen, daß diese sogar von Laien durchgeführt werden könnten. Ein weiteres Ziel ist es, die Analysen ohne und/oder nur mit einfachen Hilfsmitteln oder Gerätschaften durchführen zu können.

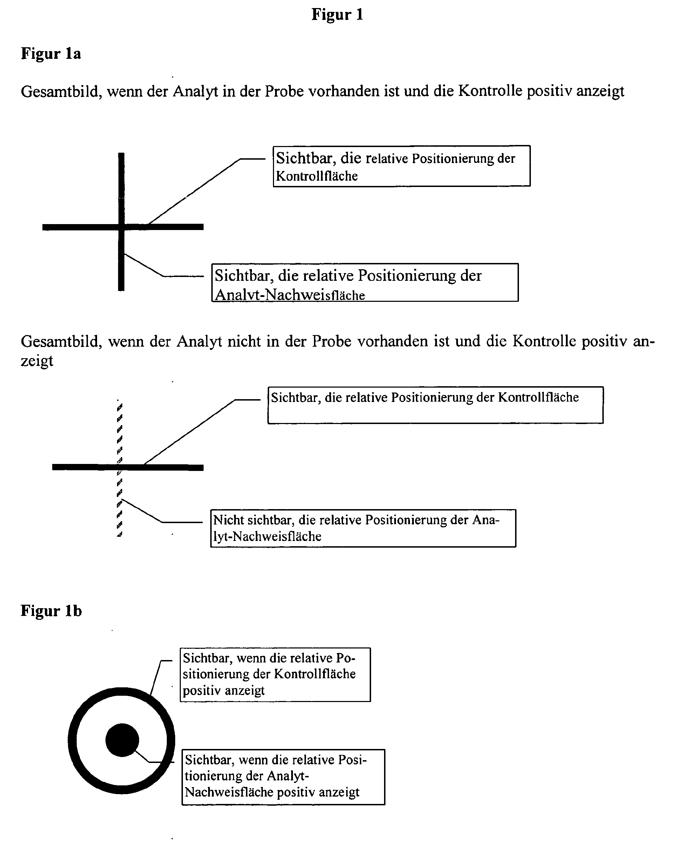

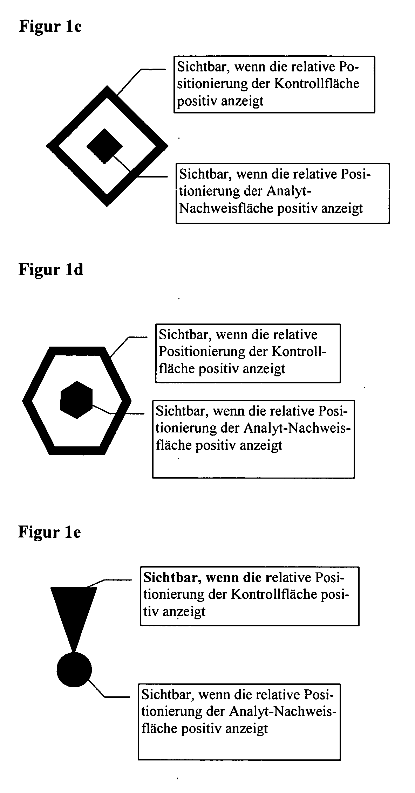

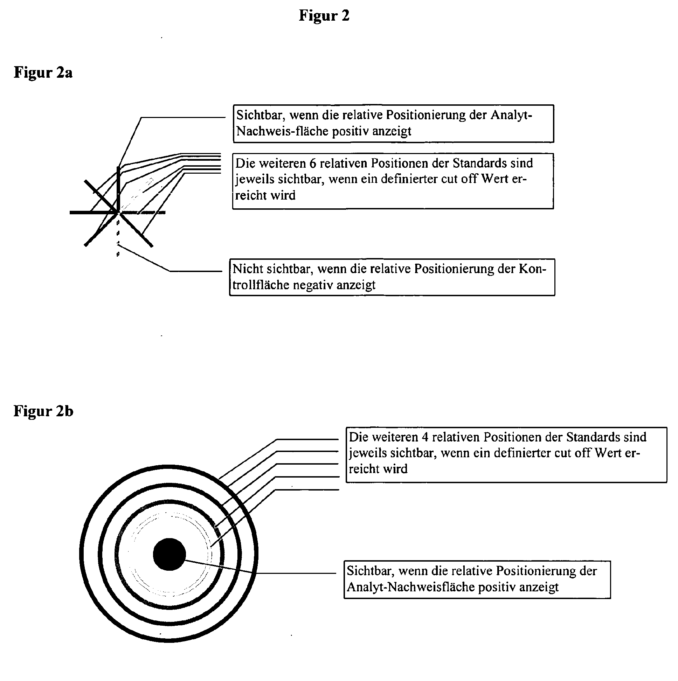

Diese Aufgabe wird erfindungsgemäß durch eine Vorrichtung, in dem auf einer Oberfläche Moleküle immobilisiert werden, welche in der Lage sind, mit den nachzuweisenden Analyten zu reagieren, so daß diese gebunden werden und in einer nachfolgenden Reaktion oder mehreren Reaktionsschritten nachgewiesen werden können, gelöst. Solche Moleküle sind z.B. Antikörper, Antigene, DNA, RNA, Enzyme, Rezeptoren, Lektine, Proteine oder Peptide. Die Immobilisierungsflächen werden derart angeordnet, daß eine positiv oder negativ Kontrolle in direktem örtlichen Zusammenhang mit dem Analyten steht. Eine Beispiel, welches den Patentumfang nicht eingrenzt, ist ein Kreis mit Punkt im Zentrum Beispiel: ⊙ (siehe Fig. 1). Der äußere Kreis ist die Positiv-Kontrolle, welche also immer erscheinen muss. Der Punkt im Zentrum erscheint nur dann, wenn der Analyt in der Probe vorhanden ist (sinnbildlich: Treffer). Die Konzentration der Positv-Kontrolle auf dem Ring, kann derart abgestimmt werden, das diese erst bei einem bestimmten Schwellenwert (cut off-Wert) sichtbar wird. Auf diese Weise wird eine visuelle Quantifizierung in jedem Rasterpunkt ermöglicht. Ist der Analyt in hoher Konzentration in der Probe vorhanden, so ist der zentrale Punkt farblich deutlich gegenüber dem äußeren Ring hervorgehoben. Ist der Analyt in geringer Konzentration, so ist der zentrale Punkt gegenüber dem äußeren Ring farblich nur schwach zu erkennen und bei negativen Resultat ist der Ring leer. Werden mehrere Ringe verwendet ist eine Standardreihe darstellbar, Beispiel: © (siehe Fig. 2). Die äußeren Ringe sind Standardkonzentrationen mit einem definiertem cut-off, der zentrale Punkt innen ist der Probenmeßpunkt. An diesem Beispiel wird sehr deutlich, daß durch eine direkte grafische Kombination von Standard (meßfläche) zu Meßpunkt (-fläche), eine erhebliches Maß an Auswertungssicherheit und höheres Maß an Information erzielt werden kann.

Die in dieser Patentschrift vorgestellte Erfindung ermöglicht somit den Aufbau eines Schnelltestverfahrens, welches universell eingesetzt werden kann, um ein breites Spektrum an Analyten in einer Probe bestimmen zu können. Ferner können parallel ein oder mehrere oder eine Vielzahl an Analyten bestimmt werden. Dies kann in einem beliebigen Raster (Matrix oder Array) durchgeführt werden, z.B. linear, oder quadratisch. Solche Anordnungen mit Flächen, welche definierte Aufgaben ob aktiv oder passiv erfüllen, finden sich schon in der frühen wissenschaftlichen Literatur aller Fachgebiete und gehören damit zum Stand des Wissens; Beispiele sind Dotblot-Arrays, Mikrotiterplatten (MTP) Koordinatensysteme. Erfindungsgemäß werden dabei über interne Standards in jedem Rasterpunkt eine Funktions-, Qualitätskontrolle und/oder Mengenbestimmungen durchgeführt, so daß ein eventueller Konzentrationsgradient der zu bestimmenden Analyten über dem Raster nicht zu Fehlinterpretationen führt. Bei entsprechender Wahl der Nachweisreaktion, z.B. Enzym Immuno Assay (EIA), Nachweis mittels Gold-, Selen-, oder Latexpartikeln gebunden an Antikörpern, kann der Test mit bloßem Auge, also rein visuell ausgewertet werden. Ferner kann der Test mit einfachen Hilfsmitteln sich auch der Nachweisverfahren wie Fluoreszenzmarkierung, elektrochemischer Detektion oder spektroskopischer Verfahren bedienen.

Weiterhin kann über die grafische Gestaltung von Standard zu Probe, eine besondere Auswertungssicherheit gewährleistet werden. Dabei werden allgemein verständliche Symbole verwendet z.B. + für positive Probe (Analyt vorhanden) und - für negative Probe (kein Analyt vorhanden). Für LFT wurde dies in der Patentschrift EP 0 421 294 B1 beschrieben, aber hier kann nur ein Analyt in der Probenflüssigkeit nachgewiesen werden und es ist keine Möglichkeit zur Quantifizierung gegeben. Im erfindungsgemäßen Test können auf diese Weise eine Vielzahl von zu bestimmenden Analyten dargestellt werden und zugleich in jedem Rasterpunkt quantifiziert werden. Ferner können auch andere Darstellungsformen gewählt werden wie Ring und innenliegender Punkt oder Stern.

Ein weiteres nicht begrenzendes Beispiel ist ein Sternsymbol (siehe Fig.2). In diesem Falle sind fünf der sechs Sternstrahlen Positiv-Kontrollen (entsprechend einer Standardreihe eines Labortestverfahrens). Diese sind derart eingestellt, daß diese jeweils einzeln ab bestimmten Konzentrationsschwellen (z.B.: aufsteigend) visuell sichtbar werden. Der Analyt wird, falls in der Probe vorhanden, entsprechend seiner Konzentration, ebenfalls sichtbar. Durch die unmittelbare örtliche Zusammenfassung von Meßpunkt (Analytpunkt) und Kontrollen (in diesem Fall einer Standardreihe), wird die sichere Durchführung einer Konzentrationsbestimmung ermöglicht. Die Genauigkeit der visuellen Ablesung ist über die Zahl der Sternstrahlen einstellbar. Die Wahl der grafischen Darstellung hängt von der späteren Anwendung ab. Wird eine reine visuelle, also mit bloßem Auge, auswertbarer Testformat angestrebt, so haben sich die Symbole + -, Kreis mit Punkt oder Stern bewährt. Wird ein Testformat angestrebt, welches mittels technischer Hilfsmittel, gleich welcher Art, auszuwerten ist, so haben sich Kreis mit zentralem Punkt und Quadrat mit zentralem Kreuz bewährt. Insbesondere können bei Auswertung mit technischen Hilfsmitteln, wie CCD-Kamera, Fluoreszenzdetektion, elektrochemischer Detektion oder andere spektroskopische Verfahren auch produktionsmäßige Fehler erkannt und bewertet werden, denn Verschleppung von Testmaterial wird durch Unschärfe zwischen den grafischen Punkten auf einfache Weise erkannt und per Computerauswertung automatisch zu bewerten. Dies ist bei allen Chip-Varianten ob DNA, RNA oder Proteinchips nicht möglich, da kein direkter, enger räumlicher Zusammenhang realisiert wurde. Werden zudem noch Oberflächen zur Immobilisierung von Molekülen eingesetzt, die glatt oder deren Poren kleine bzw. inaktiviert sind, können Probenmaterialien wie Vollblut, Kapillarblut, Serum, Plasma, Urin, Fäzes oder Proben hoher Viskosität und/oder starker Färbung ohne eine weitere Aufbereitungsmethoden direkt in das erfindungsgemäße Testverfahren eingesetzt werden. Ferner können Zellsuspensionen, Gewebebiooptate oder Lösungen aller Art eingesetzt werden.

Die erfindungsgemäße Vorrichtung ist ferner dadurch gekennzeichnet, daß es sich nicht um chromatographische Verfahren, wie den Lateral-Flow-Test (LFT), Flow-Through-Test (FTT) und auch nicht um Verfahrensmethoden wie den Agglutinations-Test (AT) oder Solid-Phase-Test (SPT) handelt, sondern um ein Tangential-Touch-Testprinzip (TTT). Bei dieser Art des erfindungsgemäßen Verfahrens reicht es aus, wenn die zu analysierende Probe mit der Immobilisierungsfläche in Kontakt kommt, dabei ist es unerheblich ob die Probe fließt, sich in Ruhe befindet oder aktiv und/oder passiv auf die Oberfläche appliziert wird. Das TTT-Prinzip unterscheidet sich insbesondere dadurch, daß es hiermit möglich wird Meßflächen und Kontrollflächen zeitlich und räumlich simultan mit der Probe in Kontakt zubringen. So ist z.B. bei allen sogenannt DIP-Stick-Verfahren oder chromatographischen Verfahren nicht möglich, da immer die Benetzung mit der Probe sequentiell erfolgt - ein DIP-Stick wird in die Probeflüssigkeit eingetaucht oder die Probe wir chromatographisch durch Materialien gesogen oder durchflossen. Auch ist es nicht notwendig die Immubilisierungsflächen derart anzuordnen, daß diese zu einer Fließrichtung optimal ausgerichtet sind (z.B.: EP 0421 294 B1, Titel: Improved self-performing immunochromatographic device).

Die Kombination von immobilisierten Molekülen auf Oberflächen, gleich welcher Art und Form oder Material, in abgegrenzten Flächen zum Nachweis von Analyten und einer zusätzlichen räumlich eng zugeordneten, grafisch abgestimmten Immobilisierungsfläche oder -Flächen zur Funktionskontrolle und/oder Quantifizierung zu jedem Analytenmesspunkt im Tangential-Touch-Test Prinzip ist bisher nicht beschrieben worden und stellt die erfindungsgemäße Vorrichtung dar.

Die erfindungsgemäße Vorrichtung wird durch ein erfindungsgemäßes Verfahren ergänzt, welches dadurch gekennzeichnet ist, daß der über immobilisierte Moleküle gebundene Analyt mittels einer nachfolgenden Reaktion oder Reaktionen bestimmt werden kann, welche nachfolgend als Nachweisreaktion bzw. Reaktionen bezeichnet werden. Die zur Durchführung benötigten Nachweisreagenzien können sich komplett auf der Oberfläche frei, z.B. als Lyophilisat oder in Lösung befinden oder teilweise bzw. ganz in getrennten Schritten zugegeben werden. Vorteil der Vorrichtung und des Verfahrens ist es, daß alle klassischen Nachweisverfahren, wie Antigen-Antikörperreaktion, markierter Antikörper oder Antigene, Biotin-Avidin, DNA oder RNA-Sonden, elektrochemische Nachweisverfahren oder spektroskopische Verfahren verwendet werden können. Die Kombination dieser Nachweisverfahren mit der erfindungsgemäßen Vorrichtung stellt das erfindungsgemäße Verfahren dar.

Die Zahl und Anordnung der kombinierten Rasterelemente ist nur durch die Anwendung oder den Anwendungszweck limitiert. Bei einer Anwendung hinsichtlich einer ausschließlichen visuellen Auswertung, ist letztlich die Auflösungsgrenze des menschlichen Auges der limitierende Faktor. Bewährt haben sich in diesem Anwendungsfall Rasterformate bis zu einer Zahl von 100. Werden technische Hilfsmittel zur Auswertung hinzugezogen, so können je nach technischem Verfahren und Aufwand bis zu 100.000 Rasterelemente ausgewertet werden.

Im Hinblick auf das oben Gesagte betrifft ein erster Aspekt der vorliegenden Erfindung somit eine Vorrichtung zum Nachweis von Molekülen oder Molekülklassen oder Molekülgemischen die dadurch gekennzeichnet, daß a) auf einer Oberfläche der Vorrichtung mindestens zwei Flächen derart chemisch oder physikalisch modifiziert sind, daß diese mit Mitteln zur Immobilisierung von Molekülen oder Molekülklassen und/oder Gemischen versehen sind, wobei eine Fläche zur Kontrolle bzw. Standardisierung, und die andere zum Nachweis eines Analyten verwendet wird, wobei b) die beiden Flächen so aufgebaut sind, daß sie im wesentlichen zum gleichen Zeitpunkt mit einer gesamten Probenmatrix, aus der Molekülen oder Molekülklassen oder Molekülgemischen bestimmt werden sollen, in Kontakt kommen (Tangential-Touch-Test Prinzip), und c) wobei beide Flächen so aufgebaut und zueinander angeordnet sind, daß sie gemeinsam ausgewertet werden, wobei sie eine optisch auslesbare grafische Anordnung bilden.

Bevorzugt ist eine erfindungsgemäße Vorrichtung zum Nachweis von Molekülen oder Molekülklassen oder Molekülgemischen die dadurch gekennzeichnet ist, daß die Flächen planar, räumlich und/oder in Körperform zueinander angeordnet sind.

Erfindungsgemäß kann die Probenmatrix aus der die oder der Analyt bestimmt werden soll flüssig, fest, gasförmig oder in physikalischen Zwischenzuständen oder Gemischen davon vorliegen.

In einem weiteren Aspekt der erfindungsgemäßen Vorrichtung zum Nachweis von Molekülen oder Molekülklassen oder Molekülgemischen ist diese dadurch gekennzeichnet, daß die Flächen in einem oder einer Vielzahl von Symbolen linear oder in einer anders angeordneten Matrix dargestellt werden.

In einem noch weiteren Aspekt der erfindungsgemäßen Vorrichtung zum Nachweis von Molekülen oder Molekülklassen oder Molekülgemischen ist diese dadurch gekennzeichnet, daß die immobilisierten Moleküle oder Molekülklassen und/oder Gemische mittels einer Nachweisreaktion visuell ohne weitere technische Hilfsmittel bestimmt werden können, wobei die verschieden Flächen farbig, schwarz oder grau getönt oder einem Gemisch aus Farben und/oder Grautönen erscheinen.

Bevorzugt ist eine erfindungsgemäße Vorrichtung zum Nachweis von Molekülen oder Molekülklassen oder Molekülgemischen, die als Gefäß mit einer oder mehreren Öffnungen ausgestaltet ist. Weiter bevorzugt ist eine erfindungsgemäße Vorrichtung zum Nachweis von Molekülen oder Molekülklassen oder Molekülgemischen nach die dadurch gekennzeichnet ist, daß sich die Flächen im Innenraum des Gefäßes befinden oder sich eine oder mehrere Flächen auf der Gefäßwandung befindet.

In einem noch weiteren Aspekt der erfindungsgemäßen Vorrichtung zum Nachweis von Molekülen oder Molekülklassen oder Molekülgemischen ist diese dadurch gekennzeichnet, daß als Symbolpaare - für negativ und + für positiv, oder Kreis für negativ und Kreis mit innen liegendem Punkt/Punkten für positiv sichtbar werden.

Gemäß einem weiteren Aspekt der erfindungsgemäßen Vorrichtung zum Nachweis von Molekülen oder Molekülklassen oder Molekülgemischen ist diese dadurch gekennzeichnet, daß die Mittel zur Immobilisierung von Molekülen oder Molekülklassen und/oder Gemischen ausgewählt sind aus der Gruppe bestehend aus Antikörper, Antigenen, DNA, RNA, Enzymen, Substraten, Rezeptoren, Liganden und Kombinationen davon.

Die Aufgabe der vorliegenden Erfindung wird weiterhin durch ein Verfahren zum Nachweis von Molekülen oder Molekülklassen oder Molekülgemischen gelöst, das umfaßt a) ein Kontakt bringen einer Probenmatrix, aus der Moleküle oder Molekülklassen oder Molekülgemische bestimmt werden sollen, mit der Oberfläche einer Vorrichtung auf solche Weise, daß diese im wesentlichen zum gleichen Zeitpunkt mit der gesamten Probenmatrix, aus der Molekülen oder Molekülklassen oder Molekülgemischen bestimmt werden sollen, in Kontakt kommt (Tangential-Touch-Test Prinzip), wobei auf der Oberfläche der Vorrichtung mindestens zwei Flächen derart chemisch oder physikalisch modifiziert sind, daß diese mit Mitteln zur Immobilisierung von Molekülen oder Molekülklassen und/oder Gemischen versehen sind, wobei eine Fläche zur Kontrolle bzw. Standardisierung, und die andere zum Nachweis eines Analyten vorgesehen ist und wobei beide Flächen so aufgebaut und zueinander angeordnet sind, daß sie gemeinsam ausgewertet werden, wobei sie eine optisch auslesbare grafische Anordnung bilden, und b) eine Auslesung und Auswertung der Flächen. Bevorzugt ist ein erfindungsgemäßes Verfahren das dadurch gekennzeichnet ist, daß die Auslesung der Flächen planar, räumlich und/oder in Körperform erfolgt.

Gemäß einem weiteren Aspekt des Verfahrens der Erfindung ist dieses dadurch gekennzeichnet, daß die verschieden Nachweisflächen farbig, schwarz oder grau getönt oder einem Gemisch aus Farben und/oder Grautönen erscheinen. Bevorzugt ist, daß die Flächen in einem oder einer Vielzahl von Symbolen linear oder in einer anders angeordneten Matrix ausgelesen werden. Weiter bevorzugt ist, daß als Symbolpaare - für negativ und + für positiv, oder Kreis für negativ und Kreis mit innen liegendem Punkt/Punkten für positiv sichtbar werden.

Gemäß eines noch weiteren Aspekts des Verfahrens der Erfindung ist dieses dadurch gekennzeichnet, daß als Symbolpaare aus mehreren ineinander liegenden Kreisen mit einem zentralen Punkt sichtbar werden, der nur im positiven Nachweisfall erscheint, und wobei jeder einzelne Kreis nur ab einem bestimmten Konzentrationswert des Analyten erscheint oder ein Stern, bei dem jeder der Strahlen ab einem bestimmten Konzentrationswert sichtbar wird, sowie im positiven Fall ein zuvor definierter Strahl erscheint oder das die einzelnen Strahlen verschiedene Analyten nachweisen und ein Strahl an einem bestimmten Konzentrationswert erscheint oder einer Kombination aus diesen Symbolpaaren.

Gemäß eines noch weiteren Aspekts des Verfahrens der Erfindung ist dieses dadurch gekennzeichnet, daß die Probenmatrix, aus der die oder der Analyt bestimmt wird, flüssig, fest, gasförmig oder in physikalischen Zwischenzuständen oder Gemischen davon vorliegt. Bevorzugt ist, daß als Probenmatrix Vollblut, Kapillarblut, Nabelschnurblut, arterielles oder venöses Vollblut, Serum, Plasma, Urin, Fäzes, Tränenflüssigkeit, Speichel, Körperschleim, gefärbte Lösungen, Lösungen mit festen Bestandteilen oder hochviskose Flüssigkeiten verwendet werden.

Weiter bevorzugt ist, daß die Probe vor, während oder geschaltet mittels Aufreinigung, Aliquotierung, Derivatisierung und/oder Isolation zur Auftragung auf die erfindungsgemäße Oberfläche vorbereitet wird.

Gemäß eines noch weiteren Aspekts des Verfahrens der Erfindung ist dieses dadurch gekennzeichnet, daß die Nachweisreaktionen von Molekülen oder Molekülklassen oder Molekülgemischen ausgewählt sind aus Farbstoff-, Radionukleotid-, Antikörper-, DNA- oder RNA-, Biotin-, Avidin- oder Enzym-Nachweisreaktionen oder Kombinationen davon.

Bevorzugt ist ein Verfahren der Erfindung" bei dem die immobilisierten Moleküle oder Molekülklassen und/oder Gemische mittels einer Nachweisreaktion visuell ohne weitere technische Hilfsmittel bestimmt werden. Jedoch kann ein alternatives Verfahren der Erfindung dadurch gekennzeichnet sein, daß für die Auslesung und/oder Auswertung technische Hilfsmittel verwendet werden, um eine visuelle Auswertung zu ermöglichen oder das Verfahren, wie zum Beispiel densitometrische Verfahren, spektroskopische - oder elektrochemische Verfahren mit der erfindungsgemäßen Auslesung und/oder Auswertung kombiniert werden. Auch kann das Verfahren der Erfindung dadurch gekennzeichnet sein, daß dieses mit Flow-Through-Tests, Agglutinations-Tests und/oder mit Solid-Phase-Tests kombiniert wird und eines, mehrere oder viele Symbolpaare umfaßt. Auch kann das Verfahren der Erfindung dadurch gekennzeichnet sein, daß dieses mit Schnelltestverfahren Lateral-Flow-Test kombiniert wird und mindestens zwei, mehrere oder viele Symbolpaare umfaßt.

Ein letzter Aspekt der vorliegenden Erfindung betrifft dann die Verwendung einer Vorrichtung der Erfindung zum Nachweis von Molekülen oder Molekülklassen bei der human-, veterinärmedizinischen oder pflanzlichen Diagnostik, der Lebensmitteldiagnostik, der Umweltdiagnostik, der forensischen Diagnostik, der Pharmakologie, der Toxikologie, bei Allergie, Autoimmun- oder Stoffwechselerkrankungen, Infektionserkrankungen, Geschlechtserkrankungen, parasitären Erkrankungen, Bestimmung von kleinen Molekülen wie Drogen, Medikamenten oder Stoffwechselprodukten, Zellmediatoren, Gewebetypisierung, Artentypisierung, Lebensmitteltypisierung, Antigentypisierung, Epitoptypisierung und DNA- oder RNA-Nachweis. Bevorzugt ist eine erfindungsgemäße Verwendung für die Diagnose unmittelbar vor, während oder nach einer therapeutischen Maßnahme.

Im Rahmen der vorliegende Erfindung sollen die folgenden Begriffe bedeuten (Liste der Definitionen sofern diese nicht schon international festgelegt sind. Bei nicht im folgenden aufgelisteten Abkürzungen werden immer die international gebräuchlichen bzw. festgelegten Standards verwendet)
- Aliquotierung: Aufteilung von Flüssigkeiten in kleinere Mengeneinheiten bzw. Volumina, z.B. Entnahme einer kleineren Menge aus einer Probe.
- Analyt: Nachzuweisendes Molekül oder Molekülgruppe
- AT: Agglutinations-Test
- Aufreinigung: Abtrennung von Flüssigkeiten, Partikeln, Substanzklassen, Zellen oder Zellbestandteilen, Verunreinigungen.
- Derivatisierung: Chemische oder physikalische Modifikation einer Substanz oder Substanzklasse
- FTT: Flow-Through-Test
- Isolierung: Trennung einer Substanz oder Substanzklasse aus einem komplexen Gemisch, gleich ob sich dieses in einer festen, gasförmigen oder flüssigen Phase oder einer Kombination befindet.
- LFT: Lateral-Flow-Test
- Nachweis: Bestimmung des Vorhandensein eines oder mehrerer des Moleküle oder Molekülgruppen mit oder ohne Quantifizierung
- Parameter: Synonym zu Analyt

- Quantifizierung: Mengenmäßige Bestimmung von Molekülen oder Molekülgruppen
- SPT: Solid-Phase-Test
- Technische Hilfsmittel: Unter technische Hilfsmittel werden alle einfachen Mittel verstanden, die zur Sichtbarmachung, Verstärkung, Modifikation, Auswertung oder deren Kombination verwendet werden. Z.B. Folien, Lichtquellen, Detektoren aller Art, chemische oder physikalische Reaktionen, welche vor während oder nach der Messung angewendet werden. Eine Brille welche nur zur Verbesserung des natürlichen Seevermögens dient, wird nicht als technisches Hilfsmittel bezeichnet.
- Visuelle Auswertung: Beurteilung eines Messergebnisses mittels ausschließlicher in Augenscheinnahme oder auch mit bloßem Auge. Dabei zählt eine Brille nicht als technisches Hilfsmittel
- Negativ: Analyt ist nicht in der Probe oder unter einem festgelegten cutoff Wert.
- Positiv: Analyt ist in der Probe und/oder über einem bestimmten festgelegt cut-off Wert.
- Matrix: Synomym eng.: Array Beliebige Anordnung und Anzahl von Flächen, welche innerhalb eines Koordinatensystems individuell identifizierbar sind, z.B.: eines klassischen Schachbrettkoordinatensystems oder MTP Koordinatensystems.
- MTP: Mikrotiterplatten
- Cut off: Konzentrationsgrenzwert ab den das Ergebnis bei vorhandenem Analyt als positiv, im Sinne einer Ergebnisbeurteilung, zu werten ist.

Das erfindungsgemäße Verfahren kann in voneinander abweichenden alternativen technischen Vorgehensweisen durchgeführt werden und in der Kombination mit klassischen Schnelltestverfahren angewendet werden.

Die Erfindung soll in den nachfolgenden Beispielen unter bezug auf die beigefügten Zeichnungen weiter erläutert werden. Die Beispiele sollen den Umfang der Erfindung nicht einschränken, sondern die Möglichkeiten des Verfahrens aufzeigen.

Fig. 1 zeigt Beispiele für grafische Symbolpaare mit einfacher Positiv-Kontrolle eines definierten cut off - Fig. 1a - Symbol "Kreuz"; Fig. 1b - Symbol "Kreis mit Punkt"; Fig. 1c - Symbol "Raute mit innerer Raute"; Fig. 1d - Symbol "Vieleck mit Vieleckzentrum"; und Fig. 1e - Symbol "Dreiecke mit Punkt"

Fig. 2 zeigt Beispiele für grafische Symbolpaare mit stufenweiser Standardreihe mit oder ohne Negativ-Kontrolle - Fig. 2a - Symbol "Stern mit Negativ-Kontrolle"; Fig. 2b - Symbol "Kreise ohne Negativ-Kontrolle";

### Beispiele

Das erfindungsgemäße Verfahren kann mit dem bekannten Schnelltestverfahren wie dem Lateral-Flow-Test (LFT), Flow-Through-Test (FTT), Agglutinations-Test (AT) oder Solid-Phase-Test (SPT) kombiniert werden. Ferner können alle optischen Verfahren, wie densitometrische Verfahren, spektroskopische - oder elektrochemische Verfahren mit dem erfindungsgemäßen Verfahren kombiniert werden. Beispiele verfahrenstechnischer Kombinationen
1. Zur Sichtbarmachung von grafisch direkt verbundenen Symbolen nach dem erfindungsgemäßen verfahren, gleich welcher Empfindlichkeitsstufe (Visuell oder mit technischen Hilfsmitteln), können Antikörper/Antigen-Reaktionen, DNA- oder RNA-Sondenreaktionen, Enzym/Substratreaktionen, Rezeptor/Ligand-Reaktionen oder Oberflächenaffinitätsreaktionen oder deren Kombinationen verwendet werden. Die Auswahl der Nachweisreaktion ist nur abhängig von dem oder den nachzuweisenden Analyten und den Bedingungen unter denen der Nachweis stattfinden soll.
2. Durchführung des Verfahrens mit der erfindungsgemäßen Vorrichtung mit einem speziellen Gefäß oder ohne ein solches.
3. Durchführung des Verfahrens mit einer zuvor, während oder nachgeschalteten durchgeführten Aufreinigung, Aliquotierung, Derivatisierung und/oder Isolation.

Die Kombination der erfindungsgemäßen Vorrichtung und mit dem Verfahren kann für viele Anwendungsgebiete, u.a. der human- oder veterinärmedizinischen Diagnostik, der Lebensmitteldiagnostik, der Umweltdiagnostik, der forensischen Diagnostik, eingesetzt werden. Im folgenden werden einige ausgewählte Anwendungsbeispiel dargestellt, welche aber den Patentumfang nicht einschränken, sie dienen lediglich zur Verdeutlichung.
1. Die Kombination aus dem erfindungsgemäßen Verfahren und der Vorrichtung kann zur Bestimmung von Patienten spezifischen Antikörper- oder Epitop-Profilen eingesetzt werden. Hier ist es notwendig, eine Vielzahl von verschiedenen spezifischen Antikörpern oder Epitopen, welche sich im Körper des Patienten befinden, simultan zu bestimmen. Dies ist bei Erkrankungen wie der Allergie, Autoimmunoder Stoffwechselerkrankungen notwendig.
2. Die Kombination aus dem erfindungsgemäßen Verfahren und der Vorrichtung kann zur Bestimmung von Bestandteilen aus Lebensmitteln eingesetzt werden. Dies kann in zwei Richtungen geschehen, zum einen die Bestimmung von Inhaltsstoffen in einem Lebensmittel und zum anderen die Bestimmung einzelner Lebensmittel in Gemischen (Fertiggerichte etc.).
3. Die Kombination aus dem erfindungsgemäßen Verfahren und der Vorrichtung kann zur Bestimmung von Infektionserkrankungen, Geschlechtserkrankungen oder parasitären Erkrankungen eingesetzt werden. Dabei können auf einem Raster sowohl keimspezifische Antigene, als auch keimspezifische Antikörper bestimmt werden.
4. Die Kombination aus dem erfindungsgemäßen Verfahren und der Vorrichtung kann zur Bestimmung von Stoffwechselerkrankungen eingesetzt werden. Hier können in einem Test verschiedene Stoffwechselenzyme oder/und deren Stoffwechselprodukte simultan bestimmt werden.
5. Die Kombination aus dem erfindungsgemäßen Verfahren und der Vorrichtung kann zur Bestimmung von kleinen Molekülen wie Drogen, Medikamenten, Zellmediatoren oder vergleichbar kleinen Substanzen verwendet werden. Diese Bestimmungen sind in der Pharmakologie, Toxikologie und der forensischen Analytik notwendig.
6. Die Kombination aus dem erfindungsgemäßen Verfahren und der Vorrichtung kann zur Bestimmung von DNA oder / und RNA Spezies eingesetzt werden. Auf diese Weise können z.B. genetisch bedingte Stoffwechselerkrankungen bestimmt oder Virus Infektionen im Frühstadium erkannt werden. Ferner können auf diese Weise Gewebetypisierungen aller Art, einschließlich Mensch, Tier, Pflanze und Pilze durchgeführt werden.

### Liste der zitierten Patentliteratur

- DE 19721151, EP 98928264.5, WO 98/53321 Titel: Streifentest zur in vitro Allergiediagnostik.
- EP 0421 294 B Titel: Improved self-performing immunochromatographic device.
- EP 1369391; WO96/10747 Titel: Device an method utilizing arrays of structures for analyte capture.
- WO 03/094716; US 2003-212316 Titel: Method an apparatus for determining blood paramters and vital signs of a patient.
- WO 02/084249 Titel: Therapeutic an diagnostic uses of antibody specificity profiles.
- WO 00/40967 Titel: Method and Device for diagnosing allergy Symptoms
- EP 0875758 B1 Immunoassay.
- WO 02/066602, EP 1350111 WO 93/10458Titel: Binding of Milk allergens to a solid phase.
- EP 1338895 Titel: High density allergen microarray
- US 6,528,325, W002/056017 Titel: Method for the visual detection of specific antibodies in human serum by the use of lateral flow assays.
- EP 1327884 Titel: Reagent test strip comprising conttrol means and timer means
- WO 97/31268 Titel: Chromatographic strip having detection andcontrol zones oriented paraell to the direction flow
- US 6,040,195 Titel: Diagnostic sanitary test strip.
- US 6,509,196, WO 01/50129 Titel: Compensation for non-spcific signals in quantitative immunoassays.

## Patentansprüche

1. Vorrichtung zum Nachweis von Molekülen oder Molekülklassen oder Molekülgemischen, **dadurch gekennzeichnet, daß**
a) auf einer Oberfläche der Vorrichtung mindestens zwei Flächen derart chemisch oder physikalisch modifiziert sind, daß diese mit Mitteln zur Immobilisierung von Molekülen oder Molekülklassen und/oder Gemischen versehen sind, wobei
eine Fläche zur Kontrolle bzw. Standardisierung, und
die andere zum Nachweis eines Analyten verwendet wird, wobei
b) die beiden Flächen so aufgebaut sind, daß sie im wesentlichen zum gleichen Zeitpunkt mit einer gesamten Probenmatrix, aus der Molekülen oder Molekülklassen oder Molekülgemischen bestimmt werden sollen, in Kontakt kommen (Tangential-Touch-Test Prinzip), und
c) wobei beide Flächen so aufgebaut und zueinander angeordnet sind, daß sie gemeinsam ausgewertet werden, wobei sie eine optisch auslesbare grafische Anordnung bilden.

2. Vorrichtung zum Nachweis von Molekülen oder Molekülklassen oder Molekülgemischen nach Anspruch 1, **dadurch gekennzeichnet, daß** die Flächen planar, räumlich und/oder in Körperform zueinander angeordnet sind.

3. Vorrichtung zum Nachweis von Molekülen oder Molekülklassen oder Molekülgemischen nach einem der Ansprüche 1 oder 2, **dadurch gekennzeichnet, daß** die Flächen in einem oder einer Vielzahl von Symbolen linear oder in einer anders angeordneten Matrix dargestellt werden.

4. Vorrichtung zum Nachweis von Molekülen oder Molekülklassen oder Molekülgemischen nach einem der Ansprüche 1 bis 4, **dadurch gekennzeichnet, daß** die immobilisierten Moleküle oder Molekülklassen und/oder Gemische mittels einer Nachweisreaktion visuell ohne weitere technische Hilfsmittel bestimmt werden können, wobei die verschieden Flächen farbig, schwarz oder grau getönt oder einem Gemisch aus Farben und/oder Grautönen erscheinen.

5. Vorrichtung zum Nachweis von Molekülen oder Molekülklassen oder Molekülgemischen nach einem der Ansprüche 1 bis 4, **dadurch gekennzeichnet, daß** dieses als Gefäß mit einer oder mehreren Öffnungen ausgestaltet ist.

6. Vorrichtung zum Nachweis von Molekülen oder Molekülklassen oder Molekülgemischen nach einem der Ansprüche 1 bis 5, **dadurch gekennzeichnet, daß** als Symbolpaare - für negativ und + für positiv, oder Kreis für negativ und Kreis mit innen liegendem Punkt/Punkten für positiv sichtbar werden.

7. Vorrichtung zum Nachweis von Molekülen oder Molekülklassen oder Molekülgemischen nach einem der Ansprüche 1 bis 6, **dadurch gekennzeichnet, daß** die Mittel zur Immobilisierung von Molekülen oder Molekülklassen und/oder Gemischen ausgewählt sind aus der Gruppe bestehend aus Antikörper, Antigenen, DNA, RNA, Enzymen, Substraten, Rezeptoren, Liganden und Kombinationen davon.

8. Verfahren zum Nachweis von Molekülen oder Molekülklassen oder Molekülgemischen, umfassend
a) ein Kontakt bringen einer Probenmatrix, aus der Moleküle oder Molekülklassen oder Molekülgemische bestimmt werden sollen, mit der Oberfläche einer Vorrichtung auf solche Weise, daß diese im wesentlichen zum gleichen Zeitpunkt mit der gesamten Probenmatrix, aus der Molekülen oder Molekülklassen oder Molekülgemischen bestimmt werden sollen, in Kontakt kommt (Tangential-Touch-Test Prinzip), wobei auf der Oberfläche der Vorrichtung mindestens zwei Flächen derart chemisch oder physikalisch modifiziert sind, daß diese mit Mitteln zur Immobilisierung von Molekülen oder Molekülklassen und/oder Gemischen versehen sind, wobei eine Fläche zur Kontrolle bzw. Standardisierung, und die andere zum Nachweis eines Analyten vorgesehen ist und wobei beide Flächen so aufgebaut und zueinander angeordnet sind, daß sie gemeinsam ausgewertet werden, wobei sie eine optisch auslesbare grafische Anordnung bilden, und
b) Auslesung und Auswertung der Flächen.

9. Verfahren nach Anspruch 8, **dadurch gekennzeichnet, daß** die Auslesung der Flächen planar, räumlich und/oder in Körperform erfolgt.

10. Verfahren nach einem der Ansprüche 8 oder 9, **dadurch gekennzeichnet, daß** als Symbolpaare - für negativ und + für positiv, oder Kreis für negativ und Kreis mit innen liegendem Punkt/Punkten für positiv sichtbar werden.

11. Verfahren nach einem der Ansprüche 8 oder 9, **dadurch gekennzeichnet, daß** als Symbolpaare aus mehreren ineinander liegenden Kreisen mit einem zentralen Punkt sichtbar werden, der nur im positiven Nachweisfall erscheint, und wobei jeder einzelne Kreis nur ab einem bestimmten Konzentrationswert des Analyten erscheint oder ein Stern, bei dem jeder der Strahlen ab einem bestimmten Konzentrationswert sichtbar wird, sowie im positiven Fall ein zuvor definierter Strahl erscheint oder das die einzelnen Strahlen verschiedene Analyten nachweisen und ein Strahl an einem bestimmten Konzentrationswert erscheint oder einer Kombination aus diesen Symbolpaaren.

12. Verfahren nach einem der Ansprüche 8 bis 11, **dadurch gekennzeichnet, daß** die Probe vor, während oder geschaltet mittels Aufreinigung, Aliquotierung, Derivatisierung und/oder Isolation zur Auftragung auf die erfindungsgemäße Oberfläche vorbereitet wird.

13. Verfahren nach einem der Ansprüche 8 bis 12, **dadurch gekennzeichnet, daß** die immobilisierten Moleküle oder Molekülklassen und/oder Gemische mittels einer Nachweisreaktion visuell ohne weitere technische Hilfsmittel bestimmt werden.

14. Verfahren nach einem der Ansprüche 8 bis 13, **dadurch gekennzeichnet, daß** als Probenmatrix Vollblut, Kapillarblut, Nabelschnurblut, arterielles oder venöses Vollblut, Serum, Plasma, Urin, Fäzes, Schleimhautflüssigkeit, Augen- bzw, Tränenflüssigkeit, gefärbte Lösungen, Lösungen mit festen Bestandteilen oder hoch viskose Flüssigkeiten, gleich welchen Aggregatzustandes verwendet wird.

15. Verwendung einer Vorrichtung nach einem der Ansprüche 1 bis 7 zum Nachweis von Molekülen oder Molekülklassen bei der human-, veterinärmedizinischen oder pflanzlichen Diagnostik, der Lebensmitteldiagnostik, der Umweltdiagnostik, der forensischen Diagnostik, der Pharmakologie, der Toxikologie, bei Allergie, Autoimmunoder Stoffwechselerkrankungen, Infektionserkrankungen, Geschlechtserkrankungen, parasitären Erkrankungen, Bestimmung von kleinen Molekülen wie Drogen, Medikamenten oder Stoffwechselprodukten, Zellmediatoren, Gewebetypisierung, Artentypisierung, Lebensmitteltypisierung, Antigentypisierung, Epitoptypisierung und DNAoder RNA-Nachweis.
